# EUROPÄISCHE PATENTSCHRIFT

(11) **EP 1 501 773 B1**
(45) Veröffentlichungstag und Bekanntmachung des Hinweises auf die Patenterteilung: **14.07.2010**
(21) Anmeldenummer: 03727390.1
(22) Anmeldetag: 29.04.2003
(51) Int. Cl.: C07C 7/08, C07C 11/08

(54) **KONTINUIERLICHES VERFAHREN ZUR GEWINNUNG VON BUTENEN AUS EINEM C4-SCHNITT**
CONTINUOUS METHOD FOR OBTAINING BUTENES FROM A C4 FRACTION
PROCEDE POUR PRODUIRE DU BUTENE EN CONTINU A PARTIR D'UNE FRACTION C SB 4 /SB

(30) Priorität: 30.04.2002 DE 10219375
(43) Veröffentlichungstag der Anmeldung: 02.02.2005
(73) Patentinhaber: BASF SE, 67056 Ludwigshafen (DE)
(72) Erfinder: ADRIAN, Till, 67240 Bobenheim-Roxheim (DE); HEIDA, Bernd, 67158 Ellerstadt (DE); KINDLER, Klaus, 67376 Harthausen (DE)
(86) Internationale Anmeldenummer: PCT/EP2003/004435
(87) Internationale Veröffentlichungsnummer: WO 2003/093202

(56) Entgegenhaltungen:
- EP-A- 0 079 679
- US-A- 4 555 312

## Beschreibung

Die Erfindung betrifft ein kontinuierliches Verfahren zur Gewinnung von Butenen aus einem C₄-Schnitt durch Extraktivdestillation mit einem selektiven Lösungsmittel.

Der Begriff C₄-Schnitt bezeichnet Gemische von Kohlenwasserstoffen mit überwiegend 4 Kohlenstoffatomen pro Molekül. C₄-Schnitte werden beispielsweise bei der Herstellung von Ethylen und/oder Propylen durch thermisches Spalten einer Petroleumfraktion wie verflüssigtes Petroleumgas, Leichtbenzin oder Gasöl erhalten. Weiterhin werden C₄-Schnitte bei der katalytischen Dehydrierung von n-Butan und/oder n-Buten erhalten. C₄-Schnitte enthalten in der Regel Butane, n-Buten, Isobuten, 1,3-Butadien, daneben geringe Mengen an sonstigen Kohlenwasserstoffen, sowie Butine, insbesondere 1-Butin (Ethylacetylen) und Butenin (Vinylacetylen). Dabei beträgt der 1,3-Butadiengehalt im allgemeinen 10 bis 80 Gew.-%, vorzugsweise 20 bis 70 Gew.-%, insbesondere 30 bis 60 Gew.-%, während der Gehalt an Vinylacetylen und Ethylacetylen im allgemeinen 5 Gew.-% nicht übersteigt.

Die Auftrennung EP-A-0 079 679 von C₄-Schnitten ist wegen der geringen Unterschiede in den relativen Flüchtigkeiten der Komponenten ein kompliziertes destillationstechnisches Problem. Daher wird die Auftrennung durch eine sogenannte Extraktivdestillation durchgeführt, d.h. eine Destillation unter Zugabe eines selektiven Lösungsmittels (auch als Extraktionsmittel bezeichnet), das einen höheren Siedepunkt als das aufzutrennende Gemisch aufweist und die Unterschiede in den relativen Flüchtigkeiten der aufzutrennenden Komponenten erhöht.

Es sind eine Vielzahl von Verfahren zur Auftrennung von C₄-Schnitten mittels Extraktivdestillation unter Verwendung von selektiven Lösungsmitteln bekannt. Ihnen ist gemeinsam, dass sich durch Gegenstromführung des aufzutrennenden C₄-Schnittes in Dampfform mit dem flüssigen selektiven Lösungsmittel bei geeigneten thermodynamischen Bedingungen, in der Regel bei niedrigen Temperaturen, häufig bei Raumtemperatur oder bei geringfügig erhöhter Temperatur und bei Normaldruck das selektive Lösungsmittel mit den Komponenten aus dem C₄-Schnitt belädt, zu denen es eine höhere Affinität hat, d.h. mit ungesättigten oder mehrfach ungesättigten Komponenten, wogegen die gesättigten Komponenten in der Dampfphase verbleiben und als Kopfstrom abgezogen werden. Aus dem beladenen Lösungsmittelstrom werden anschließend unter geeigneten thermodynamischen Bedingungen, d.h. bei höherer Temperatur und/oder niedrigerem Druck gegenüber dem ersten Verfahrensschritt in einem oder mehreren weiteren Verfahrensschritten die ungesättigten oder mehrfach ungesättigten Komponenten fraktioniert freigesetzt, d.h. aus dem selektiven Lösungsmittel ausgegast. Das entgaste Lösungsmittel wird, nach Abkühlung desselben, in der Regel über einen Wärmeverbund, wobei die Wärme zur Temperaturerhöhung des der Ausgasung zuzuführenden Feedstroms genutzt wird, in den ersten Verfahrensschritt, d.h. zur Extraktivdestillation des C₄-Schnittes recycliert. Derartige Verfahren sind beispielsweise aus DE-A 198 188 10 oder DE-A 27 24 365 bekannt.

Demgegenüber war es Aufgabe der vorliegenden Erfindung, ein Verfahren zur Gewinnung von Butenen aus einem C₄-Schnitt durch Extraktivdestillation mit einem selektiven Lösungsmittel bereitzustellen, das besonders effizient und wirtschaftlich ist. Insbesondere sollen bei diesem Verfahren die benötigten Energiemengen und die Investitionskosten gering sein.

Die Lösung geht aus von einem kontinuierlichen Verfahren zur Gewinnung von Butenen aus einem Butane, Butene und gegebenenfalls Spuren sonstiger Kohlenwasserstoffe enthaltenden C₄-Schnitt durch Extraktivdestillation mit einem selektiven Lösungsmittel, wobei der C₄-Schnitt in einer ersten Verfahrensstufe I in einer Waschzone, der der C₄-Schnitt gasförmig oder flüssig und das selektive Lösungsmittel flüssig oberhalb der Zuführung des C₄-Schnittes zugeführt werden, in einen die Butane enthaltenden Kopfstrom und einen Sumpfstrom, enthaltend das mit den Butenen und gegebenenfalls Spuren sonstiger Kohlenwasserstoffen beladene selektive Lösungsmittel aufgetrennt wird, und wobei der Sumpfstrom in einer zweiten Verfahrensstufe II in einer Ausgaserzone, der über einen Sumpfverdampfer Energie zugeführt wird, bei erhöhter Temperatur und/oder erniedrigtem Druck gegenüber der Waschzone in einen die Butene und gegebenenfalls Spuren sonstiger Kohlenwasserstoffe enthaltenden Kopfstrom und einen das selektive Lösungsmittel enthaltenden Sumpfstrom aufgetrennt wird, wobei die Wärme des Sumpfstroms aus der Ausgaserzone zur Temperaturerhöhung in der Ausgaserzone genutzt wird.

Die Erfindung ist **dadurch gekennzeichnet, dass** aus der Ausgaserzone von einer Trennstufe, die eine oder mehrere Trennstufen unterhalb der Zuführung des Sumpfstroms aus der Waschzone liegt, die Flüssigkeit oder ein Teilstrom der Flüssigkeit aus der Ausgaserzone abgezogen, durch indirekten Wärmeaustausch mit dem heißen Sumpfstrom aus der Ausgaserzone erwärmt und/oder verdampft und auf derselben Trennstufe oder oberhalb derselben in die Ausgaserzone zurückgeführt wird, wobei die Trennstufe, von der die Flüssigkeit oder der Flüssigkeitsteilstrom abgezogen wird, dergestalt gewählt wird, dass der Gesamtenergiebedarf in den Verfahrensstufen I und II minimal ist.

Das vorliegende Verfahren ist grundsätzlich auf jeden C₄-Schnitt anwendbar, besonders vorteilhaft sind jedoch C₄-Schnitte als Ausgangsgemisch einsetzbar, die einen relativ hohen Anteil an Butenen aufweisen.

Als Spuren sonstiger Kohlenwasserstoffe werden vorliegend Gewichtsanteile sonstige Kohlenwasserstoffe verstanden, die im nachfolgenden Einsatz der aus dem C₄-Schnitt gewonnenen Produkte nicht spezifikationsschädlich sind.

Vorteilhaft einsetzbar sind beispielsweise C₄-Schnitte aus der Erdölraffinerie, aus sogenannten FCC-Crackern (Fluidized Catalytic Cracking), die in der Regel eine Zusammensetzung von 20 bis 70 Gew.-% an Butanen, 30 bis 80 Gew.-% an Butenen, Rest sonstige C₃-C₅-Kohlenwasserstoffe aufweisen, besonders bevorzugt C₄-Schnitte mit 42 Gew.-% Butanen, 56 Gew.-% Butenen und 2 Gew.-% sonstigen C₃-C₅-Kohlenwasserstoffen.

Ein typischer C₄-Schnitt aus einem FCC-Cracker weist die folgende Zusammensetzung in Gew.-% auf:

| | |
|---|---|
| Propan | 0,3 |
| Propen | 1,2 |
| n-Butan | 12 |
| i-Butan | 30 |
| 1-Buten | 14 |
| i-Buten | 10 |
| trans-Buten-2 | 15,5 |
| cis-Buten-2 | 16,5 |
| 1,3-Butadien | 0,5. |

Vorteilhaft kann als C₄-Schnitt für das vorliegende Verfahren auch sogenanntes Raffinat 1 aus einer Butadienanlage eingesetzt werden, bevorzugt unmittelbar, ohne weitere Zwischenbehandlung.

In Butadienanlagen wird 1,3-Butadien aus dieses enthaltenden C₄-Schnitten gewonnen, wobei die eingesetzten C₄-Schnitte typischerweise Zusammensetzungen in Gew.-% in den nachstehenden Bereichen aufweisen:

| | |
|---|---|
| 1,3-Butadien | 10 bis 80 |
| Butene | 10 bis 60 |
| Butane | 5 bis 40 |
| sonstige C₄-Kohlenwasserstoffe und | 0,1 bis 5 |
| sonstige Kohlenwasserstoffe, insbesondere | |
| C₃- und C₅-Kohlenwasserstoffe | 0 bis maximal 5. |

In Butadienanlagen wird der aufzutrennende C₄-Schnitt zunächst gasförmig mit dem flüssigen selektiven Lösungsmittel in einer Extraktionszone im Gegenstrom in Kontakt gebracht, wobei das 1,3-Butadien sowie weitere Kohlenwasserstoffe, für die das selektive Lösungsmittel eine höhere Affinität als für 1,3-Butadien hat, vom selektiven Lösungsmittel im wesentlichen vollständig absorbiert werden, die Komponenten, für die das selektive Lösungsmittel eine geringere Affinität hat, insbesondere die Butane und die Butene jedoch im wesentlichen in der Gasphase verbleiben. Diese Gasphase wird als Kopfstrom abgezogen und häufig als Raffinat 1 bezeichnet. Im Verfahren der DE 198 188 10 ist das Raffinat 1 der in den Figuren 1 und 2 mit Gbc bezeichnete Kopfstrom der Extraktivdestillationskolonne EI.

Im Verfahren der DE-A 27 24 365 ist das Raffinat 1 der Kopfstrom aus dem sogenannten Hauptwascher.

Eine beispielhafte Zusammensetzung für Raffinat 1 in Gew.-% ist nachstehend aufgeführt:

| | |
|---|---|
| n-Butan | 17 |
| i-Butan | 6 |
| 1-Buten | 29 |
| i-Buten | 36 |
| trans-Buten-2 | 6 |
| cis-Buten-2 | 6 |
| 1,3-Butadien | ≤0,01 |

Für die vorliegende Trennaufgabe sind selektive Lösungsmittel geeignet, deren Affinität zu Kohlenwasserstoffen mit Einfachbindungen in Richtung zu Kohlenwasserstoffen mit Doppelbindungen und weiter zu konjugierten Doppelbindungen und Dreifachbindungen zunimmt, bevorzugt dipolare, besonderes bevorzugt dipolar-aprotische Lösungsmittel. Aus apparatetechnischen Gründen werden wenig oder nicht korrosive Substanzen bevorzugt.

Geeignete selektive Lösungsmittel für das erfindungsgemäße Verfahren sind zum Beispiel Butyrolacton, Nitrile wie Acetonitril, Propionitril, Methoxypropionitril, Ketone wie Aceton, Furfurol, N-alkylsubstituierte niedere aliphatische Säureamide, wie Dimethylformamid, Diethylformamid, Dimethylacetamid, Diethylacetamid, N-Formylmorpholin, N-alkylsubstituierte cyclische Säureamide (Lactame) wie N-Alkylpyrrolidone, insbesondere N-Methylpyrrolidon. Im allgemeinen werden alkylsubstituierte niedere aliphatische Säureamide oder N-alkylsubstituierte cyclische Säureamide verwendet. Besonders vorteilhaft sind Dimethylformamid, Acetonitril, Furfurol und insbesondere N-Methylpyrrolidon.

Es können jedoch auch Mischungen dieser Lösungsmittel untereinander, zum Beispiel von N-Methylpyrrolidon mit Acetonitril, Mischungen dieser Lösungsmittel mit Colösungsmitteln wie Wasser und/oder tert.-Butylether, zum Beispiel Methyl-tert.-butylether, Ethyl-tert.-butylether, Propyl-tert.-butylether, n- oder iso-Butyl-tert.-butylether eingesetzt werden.

Besonders geeignet ist N-Methylpyrrolidon, vorliegend abgekürzt als NMP bezeichnet, bevorzugt in wäßriger Lösung, vorteilhaft mit 0 bis 20 Gew.-% Wasser, insbesondere mit 7 bis 10 Gew.-% Wasser, besonders bevorzugt mit 8,3 Gew.-% Wasser.

### Verfahrensstufe I

In Verfahrensstufe I wird ein C₄-Schnitt in einer Waschzone einer Extraktivdestillation unterworfen, indem der Waschzone der C₄-Schnitt gasförmig oder flüssig, bevorzugt gasförmig, das selektive Lösungsmittel flüssig oberhalb der Zufuhrung des C₄-Schnittes zugeführt werden. Hierbei findet im Gegenstrom von C₄-Schnitt und Lösungsmittel eine Auftrennung des C₄-Schnittes in einen die gesättigten Komponenten, d.h. die Komponenten, zu denen das selektive Lösungsmittel eine geringere Affinität hat, überwiegend Butane, enthaltenden Kopfstrom und einen Sumpfstrom statt, der das mit Komponenten, zu denen das selektive Lösungsmittel eine höhere Affinität gegenüber den Butanen aufweist, überwiegend Butene und gegebenenfalls weitere Kohlenwasserstoffe beladene Lösungsmittel enthält. Bevorzugt führt man den C₄-Schnitt der Waschzone gasförmig, im unteren Bereich derselben, zu.

Die Waschzone ist in der Regel als Kolonne ausgebildet. Es gibt grundsätzlich keine Einschränkungen bezüglich der einsetzbaren trennwirksamen Einbauten derselben: es können gleichermaßen Böden, Füllkörper oder strukurierte Packungen eingesetzt werden. Die Kolonne weist vorteilhafterweise 10 bis 80, bevorzugt 20 bis 30 theoretische Trennstufen, insbesondere 26 theoretische Trennstufen auf.

Oberhalb der Zuführung für das selektive Lösungsmittel im oberen Bereich der Kolonne ist bevorzugt eine 3 bis 5 Böden umfassende Rückwaschzone vorgesehen, in der mittels des am Kopf der Kolonne kondensierten Rücklaufs Restanteile an selektivem Lösungsmittel ausgewaschen werden.

Der Kolonnendruck in der Waschzone ist abhängig von der Temperatur des Kühlmediums im Kondensator am Kolonnenkopf (Brunnenwasser, Flusswasser, Meerwasser, Kältemittel wie Flüssigpropylen, Flüssigammoniak oder Sole). Er liegt in der Regel zwischen 1 und 15, häufig zwischen 2 und 10, vorzugsweise bei 5, 4 bar. Die Temperatur in der Kolonne wird, ausgehend von den oben genannten Druckwerten, dergestalt bestimmt, dass geeignete thermodynamische Bedingungen gegeben sind, bei denen sich das selektive Lösungsmittel mit den Komponenten aus dem C₄-Schnitt belädt, zu denen es eine größere Affinität als zu den Butanen aufweist, wogegen die Butane aus dem C₄-Schnitt in der Gasphase verbleiben. Typischerweise liegt die Temperatur am Kopf der Kolonne im Bereich von etwa 30 bis 60°C.

### Verfahrensstufe II

Der Sumpfstrom aus der Waschzone wird in Verfahrensstufe II in einer Ausgaserzone bei erhöhter Temperatur und gegebenenfalls erniedrigtem Druck gegenüber der Waschzone in einen die Butene und gegebenenfalls Spuren sonstiger Kohlenwasserstoffe enthaltenden Kopfstrom und einen das selektive Lösungsmittel enthaltenden Sumpfstrom aufgetrennt. Hierbei wird zur Temperaturerhöhung eines aus der Ausgaserzone abgezogenen Flüssigkeitsstroms die Wärme des Sumpfstroms aus der Ausgaserzone durch indirekten Wärmeaustausch genutzt.

In der Ausgaserzone müssen die thermodynamischen Bedingungen dergestalt eingestellt werden, dass die Ausgasung der Kohlenwasserstoffe aus dem selektiven Lösungsmittel, insbesondere die Ausgasung der Butene und gegebenenfalls weiterer C₃-C₅-Kohlenwasserstoffe erfolgt. Hierfür sind in der Regel, sofern als selektives Lösungsmittel NMP mit etwa 7 bis 10 Gew.-% Wasser eingesetzt wird, Sumpftemperaturen im Bereich von 150 bis 160°C und Drücke im Bereich von Normaldruck bis 10 bar absolut, bevorzugt von 1,5 bar absolut, erforderlich.

Bezüglich der apparativen Ausgestaltung handelt es sich bei der Ausgaserzone, wie auch bei der Waschzone, um eine Kolonne, die grundsätzlich mit jeder Art von trennwirksamen Einbauten ausgestattet sein kann. Bevorzugt werden trennwirksame Einbauten eingesetzt, die wenig verschmutzungsanfällig, bzw. leicht zu reinigen sind, insbesondere Böden.

Die Kolonne weist bevorzugt 1 bis 30 theroretische Trennstufen, insbesondere 2 bis 8, besonders bevorzugt vier theoretische Trennstufen auf.

Analog zur Waschzone sind auch in der Ausgaserzone bevorzugt oberhalb der Zuführung des Feedstroms Rückwaschböden für mit dem Dampfstrom mitgerissenes selektives Lösungsmittel vorgesehen, in der Regel 3 bis 5 Böden.

Als Sumpfstrom wird aus der Ausgaserzone A heißes selektives Lösungsmittel abgezogen. Dieses wird im Wärmeverbund, d.h. indem sein Wärmeinhalt innerhalb des Verfahrens genutzt wird, abgekühlt und in die Verfahrensstufe I, d.h. in die Waschzone recycliert.

Erfindungsgemäß wird durch eine besondere Verfahrensführung die Wärme des heißen Sumpfstroms aus der Ausgaserzone A besonders effizient genutzt, so dass der Gesamtenergiebedarf für das Verfahren minimiert wird.

Hierzu wird die Flüssigkeit oder ein Teilstrom der Flüssigkeit von einer Trennstufe aus der Ausgaserzone A abgezogen, die eine oder mehrere Trennstufen unterhalb der Zuführung des Feedstroms zur Ausgaserzone liegt, durch indirekten Wärmetausch mit dem heißen Sumpfstrom aus der Ausgaserzone erwärmt und/oder verdampft und auf gleicher Stufe, von der der Strom abgezogen wurde, in die Ausgaserzone zurückgeführt.

Bevorzugt kann die Wärme des Sumpfstroms aus der Ausgaserzone A zusätzlich in der Waschzone genutzt werden, indem man aus der Waschzone von einer Trennstufe, die eine oder mehrere Trennstufen unterhalb der Zuführung des Stromes des selektiven Lösungsmittels, bevorzugt unterhalb der Zuführung des C₄-Schnittes liegt, die Flüssigkeit oder einen Teilstrom der Flüssigkeit abzieht, durch indirekten Wärmeaustausch mit dem heißen Sumpfstrom aus der Ausgaserzone erwärmt und/oder verdampft und auf derselben Trennstufe oder oberhalb derselben in die Waschzone zurückführt, wobei man die Trennstufe, von der man die Flüssigkeit oder den Flüssigkeitsteilstrom abzieht, dergestalt wählt, dass der Gesamtenergiebedarf in den Verfahrensstufen I und II minimal ist.

In einer bevorzugten Verfahrensvariante wird der abgezogene Flüssigkeitsstrom oder -teilstrom einer Entspannungsverdampfung unterworfen unter Erhalt einer dampfförmigen und einer flüssigen Phase und anschließend die dampfförmige und die flüssige Phase auf dieselbe Stufe zurückgeführt, von der der Flüssigkeitsstrom oder -teilstrom abgezogen wurde oder der dampfförmige Anteil des abgezogenen Flüssigkeitsstromes oder -teilstromes wird eine oder mehrere Stufen oberhalb der Stufe zurückgeführt, von der der Flüssigkeitsstrom oder -teilstrom abgezogen wurde.

Die Erfinder haben erkannt, dass es für jede Ausgaserzone, in Abhängigkeit von der Feedzusammensetzung, den Temperatur- und Druckbedingungen, der Trennstufenzahl sowie der vorgegebenen Spezifikation für das als Kopfstrom abgezogene Wertprodukt jeweils eine Trennstufe gibt, auf der der indirekte Wärmetausch mit dem heißen Sumpfstrom aus der Ausgaserzone am vorteilhaftesten ist, weil hierbei von außen dem Sumpfverdampfer der Ausgaserzone die geringste Energiemenge zugeführt werden muß, d.h. dass der Gesamtenergiebedarf in den Verfahrensstufen I und II minimal ist. Wird die Flüssigkeit von tiefer gelegenen Trennstufen abgezogen, so ist, infolge des Temperaturprofils in der Ausgaserzone, die Temperaturdifferenz zum Sumpfstrom gering und es kann somit nur wenig Wärme übertragen werden. Wird die Flüssigkeit dagegen von höher gelegenen Trennstufen abgezogen, so gilt folgende Überlegung: Die größte Wärmemenge kann, aufgrund der größten Temperaturdifferenz, zwischen dem Feedstrom zur Ausgaserzone und dem heißen Sumpfstrom übertragen werden. Dies ist jedoch ebenfalls nicht die wirtschaftlichste Nutzung der Energie des Sumpfstroms, da hier mehr Energie eingetragen wird, als für die Trennaufgabe an dieser Stelle notwendig ist. Dieses Übermaß an Energieangebot muß entweder über ein unnötig hohes Rücklaufverhältnis am Kondensator am Kopf der Ausgaserzone oder über einen zusätzlichen Kühler vernichtet werden.

Bevorzugt kann die Wärme des heißen Sumpfstroms aus der Ausgaserzone zusätzlich zum indirekten Wärmeaustausch mit aus der Waschzone I abgezogener Flüssigkeit genutzt werden.

Sofern das heiße Lösungsmittel durch indirekten Wärmetausch mit aus der Ausgaserzone abgezogener Flüssigkeit noch nicht genügend abgekühlt ist, um in die Extraktionszone recycliert werden zu können, kann der noch verfügbare Wärmeinhalt an einer anderen Stelle im Verfahren, bevorzugt im Sumpfverdampfer der Extraktionszone der Verfahrensstufe I, genutzt werden.

In einer bevorzugten Verfahrensvariante sind die Waschzone und Ausgaserzone in einer einzigen Kolonne angeordnet. Dadurch sind die Investitions- und Betriebskosten deutlich niedriger und die Anlage weist eine erhöhte Betriebssicherheit auf.

Die Erfindung wird im folgenden anhand einer Zeichnung sowie von Ausführungsbeispielen näher verdeutlicht.

Es zeigen im einzelnen:
- Figur 1: die schematische Darstellung einer bevorzugten Anlage zur Durchführung des erfindungsgemäßen Verfahrens,
- Figur 2: die schematische Darstellung einer weiter bevorzugten Anlage, wobei die Waschzone und die Ausgaserzone in einer einzigen Kolonne zusammengefaßt sind.

In den Figuren bezeichnen gleiche Bezugszeichen gleiche oder entsprechende Merkmale.

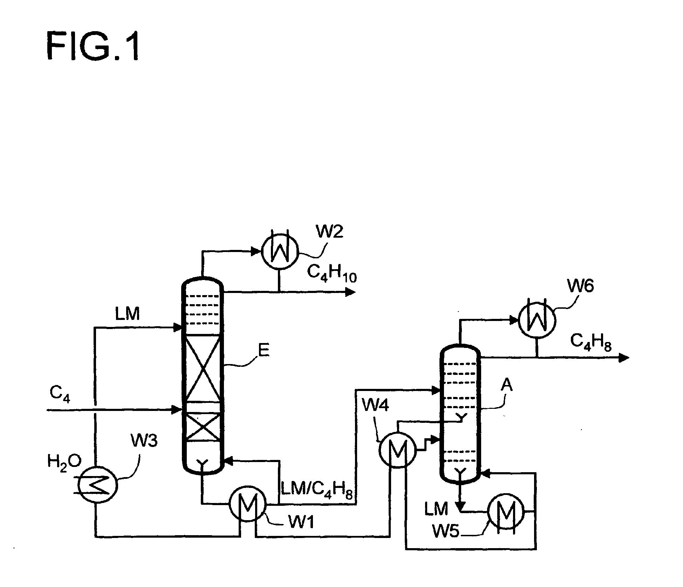

Figur 1 zeigt eine Waschzone E zur Extraktivdestillation, die als Kolonne ausgebildet ist, mit Zuführung des flüssigen Lösungsmittels LM im oberen Kolonnenbereich und Zuführung des gasförmigen C₄-Schnittes, Strom C₄, im unteren Kolonnenbereich. Aus der Kolonne wird ein überwiegend die Butane enthaltender Kopfstrom, C₄H₁₀, abgezogen und ein Sumpfstrom, der insbesondere mit Butenen und Spuren sonstiger Kohlenwasserstoffen beladenes Lösungsmittel enthält, Strom LM/C₄H₈. Der Kopfstrom wird in einem Kondensator W2 am Kolonnenkopf kondensiert. Bevorzugt wird ein Teil desselben als Rücklauf wieder auf den Kolonnenkopf aufgegeben. Am Kolonnensumpf ist ein Wärmetauscher W1 angeordnet. Es ist möglich, zwecks geeigneter Einstellung der Temperatur des selektiven Lösungsmittels LM, einen vorzugsweise mit Wasser betriebenen Wärmetauscher W3 vorzusehen. Der Sumpfstrom LM/C₄H₈ aus der Waschzone E wird eine Ausgaserzone A, im oberen Bereich derselben, aufgegeben. Der Ausgaserzone A wird über den Sumpfverdampfer W5 von außen Energie zugeführt. Der Kopfstrom aus der Ausgaserzone A wird im Kondensator W6 am Kolonnenkopf kondensiert, teilweise als Rücklauf wieder auf den Kopf der Kolonne aufgegeben und im übrigen als Wertprodukt, Strom C₄H₈, der überwiegend Butene enthält, abgezogen. Der heiße Sumpfstrom LM aus der Ausgaserzone A, der überwiegend das Lösungsmittel enthält, gibt einen Teil seines Wärmeinhalts im Wärmetauscher W4 durch indirekten Wärmetausch an die Flüssigkeit ab, die von einer Trennstufe, die unterhalb des Feedstroms liegt, aus der Ausgaserzone A abgezogen und nach Erwärmung der Ausgaserzone A derselben erneut zugeführt wird.

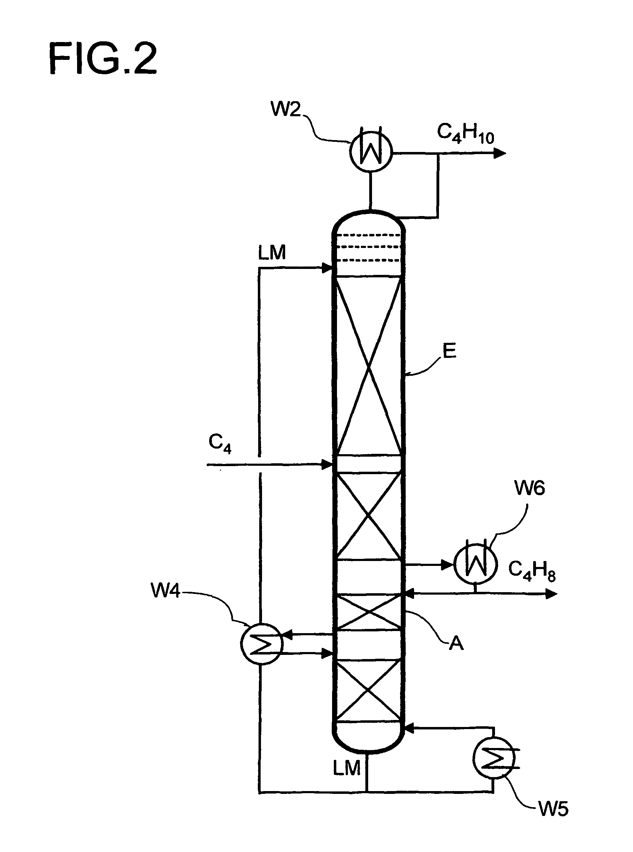

Figur 2 zeigt die schematische Darstellung einer Anlage, in der Waschzone E und Ausgaserzone A in einer einzigen Kolonne angeordnet sind.

Die im oberen Abschnitt einer Kolonne, der als Waschzone E ausgebildet ist, wird im oberen Bereich desselben flüssiges Lösungsmittel LM und im unteren Bereich desselben gasförmiger C₄-Schnitt, Strom C₄, zugeführt. Aus der Kolonne wird ein überwiegend die Butane enthaltender Kopfstrom, C₄H₁₀, abgezogen, in einem Kondensator W2 am Kolonnenkopf kondensiert und ein Teil des Kondensats als Rücklauf wieder auf den Kolonnenkopf aufgegeben. Die Flüssigkeit aus dem unteren Bereich der Waschzone E fließt in den unteren Abschnitt der Kolonne, der die Ausgaserzone A darstellt, ab.

Der Ausgaserzone A wird über den Sumpfverdampfer W5 von außen Energie zugeführt. Aus dem oberen Bereich der Ausgaserzone wird ein Strom abgezogen, in einem Kondensator W6 kondensiert, teilweise als Rücklauf wieder auf die Ausgaserzone A aufgegeben und im übrigen als Wertprodukt, Strom C₄H₈, der überwiegend Butene enthält, abgezogen. Der heiße Sumpfstrom LM aus der Ausgaserzone A, der überwiegend das Lösungsmittel enthält, gibt einen Teil seines Wärmeinhalts im Wärmetauscher W4 durch indirekten Wärmetausch an die Flüssigkeit ab, die aus der Ausgaserzone A abgezogen und nach Erwärmung der Ausgaserzone A erneut zugeführt wird.

Als trennwirksame Einbauten sind übliche Böden, Packungen, Füllkörper und ähnliches einsetzbar.

Die Erfindung wird im folgenden anhand eines Ausführungsbeispiels näher erläutert:
In einer Anlage wie in Figur 1 schematisch dargestellt, mit 30 theoretischen Trennstufen in einer Waschzone E wurde ein gasförmiger C₄-Schnitt, Strom C₄, von 13666 kg/h mit nachstehender Zusammensetzung auf die 9. theoretische Trennstufe, bei Zählung der Trennstufen von unten nach oben in der Kolonne, sowie ein flüssiger Lösungsmittelstrom LM, mit der nachstehend aufgeführten Zusammensetzung, jeweils in Gew.-%, auf die 27. theoretische Trennstufe zugeführt.

Zusammensetzung des Stromes C₄:

| | |
|---|---|
| n-Butan | 17,1 |
| i-Butan | 6,4 |
| n-Buten | 27,8 |
| i-Buten | 33,8 |
| trans-Buten-2 | 8,6 |
| cis-Buten-2 | 6,23 |
| 1,3-Butadien | 0,07 |

Zusammensetzung des Stromes LM:

| | |
|---|---|
| NMP | 91,7 |
| Wasser | 8,3 |

Oberhalb der Zuführung des Lösungsmittelstromes LM waren in der Kolonne 3 theoretische Rückwaschböden angeordnet. Die Temperatur des Stromes C₄ betrug 41,7°C, die Temperatur des Stromes LM 34°C und der Druck am Kolonnenkopf 4,05 bar.

Der Kopfstrom der Waschzone E wurde in einem Wärmetauscher W2 kondensiert, teilweise als Rücklauf wieder auf den Kolonnenkopf aufgegeben und im übrigen als Strom C₄H₁₀ abgezogen. Der Strom C₄H₁₀ enthielt vorliegend zu 95% Butane, d.h. 25,6 Gew.-% n-Butan, 69,4 Gew.-% i-Butan, Rest Verunreinigungen, überwiegend n-Buten, trans-Buten-2 und Wasser.

Der Sumpfstrom der Waschzone E, LM/C₄H₈ enthielt insbesondere mit Butenen und Spuren sonstiger Kohlenwasserstoffen beladenes Lösungsmittel, mit folgender beispielhafter Zusammensetzung in Gew.-%:

| | |
|---|---|
| n-Butan | 0,15 |
| i-Butan | 0,30 |
| n-Buten | 1,7 |
| i-Buten | 2,1 |
| trans-Buten-2 | 0,53 |
| cis-Buten-2 | 0,39 |
| Wasser | 7,8 |
| NMP | 87,3 |

Dieser Strom LM/C₄H₈ wurde als Feedstrom mit einer Temperatur von 55,4°C auf die vierte, d.h. oberste theoretische Trennstufe einer Ausgaserzone A aufgegeben. Der Kopfstrom der Ausgaserzone A wurde in einem Kondensator W6 kondensiert, teilweise als Rücklauf wieder auf die Ausgaserzone A aufgegeben und im übrigen als überwiegend Butene enthaltender Strom C₄H₈ mit der nachstehend aufgeführten Zusammensetzung abgezogen.

Zusammensetzung des Stromes C₄H₈ in Gew.-%:

| | |
|---|---|
| n-Buten | 32,5 |
| i-Buten | 40,0 |
| trans-Buten-2 | 10,1 |
| cis-Buten-2 | 7,3, |

Rest Verunreinigungen.

Der Ausgaserzone A wird über den Sumpfverdampfer W5 Energie von außen zugeführt. Der heiße Sumpfstrom LM aus der Ausgaserzone A, der überwiegend das Lösungsmittel enthielt, gab einen Teil seines Wärmeinhalts im Wärmetauscher W4 durch indirekten Wärmetausch an die Flüssigkeit ab, die von jeweils unterschiedlichen Trennstufen aus der Ausgaserzone A abgezogen und nach Erwärmung der Ausgaserzone A erneut zugeführt wurden. Beispielhaft wurden jeweils Flüssigkeitsströme von der ersten, zweiten, dritten bzw. vierten theoretischen Trennstufe der Ausgaserzone A abgezogen und durch Wärmeintegration, d.h. durch indirekten Wärmetausch mit dem heißen, aus dem Sumpf der Ausgaserzone A abgezogenen Lösungsmittelstrom LM erwärmt und erneut derselben theoretischen Trennstufe zugeführt.

Dabei ergab sich folgender Energiebedarf für die Zuführung zum Sumpfverdampfer W5 der Ausgaserzone A:

| Ort der Wärmeintegration (theoretische Trennstufe) | Energiebedarf in Megawatt |
|---|---|
| 1 | 10,7 |
| 2 | 7,0 |
| 3 | 6,2 |
| 4 | 6,4 |
| Feedstrom | 6,9 |

Die Versuche zeigen, daß der Wärmebedarf für die Anlage, d.h. die von außen zugeführte Energie, minimiert werden kann, sofern die Wärmeintegration auf der geeigneten theoretischen Trennstufe, vorliegend die dritte theoretische Trennstufe, die eine Stufe unterhalb der Zuführung des Feedstroms liegt, durchgeführt wird. Ungünstig ist ebenfalls eine Wärmeintegration in den Feedstrom.

## Patentansprüche

1. Kontinuierliches Verfahren zur Gewinnung von Butenen aus einem Butane, Butene und gegebenenfalls Spuren sonstiger Kohlenwasserstoffe enthaltenden C₄-Schnitt durch Extraktivdestillation mit einem selektiven Lösungsmittel (LM), wobei
in einer ersten Verfahrensstufe I in einer Waschzone (E), der der C₄-Schnitt (C₄) gasförmig oder flüssig und das selektive Lösungsmittel (LM) flüssig oberhalb der Zuführung des C₄-Schnittes (C₄) zugeführt werden, der C₄-Schnitt in einen die Butane enthaltenden Kopfstrom (C₄H₁₀) und einen Sumpfstrom (LM/C₄H₈), enthaltend das mit den Butenen und gegebenenfalls den Spuren sonstiger Kohlenwasserstoffe beladene selektive Lösungsmittel aufgetrennt wird, und wobei der Sumpfstrom (LM/C₄H₈)
in einer zweiten Verfahrensstufe II in einer Ausgaserzone (A), der über einen Sumpfverdampfer (W5) Energie zugeführt wird, bei erhöhter Temperatur und gegebenenfalls erniedrigtem Druck gegenüber der Waschzone (E) in einen die Butene und gegebenenfalls Spuren sonstiger Kohlenwasserstoffe enthaltenden Kopfstrom (C₄H₈) und einen das selektive Lösungsmittel enthaltenden Sumpfstrom (LM) aufgetrennt wird, wobei die Wärme des Sumpfstroms aus der Ausgaserzone (A) zur Temperaturerhöhung in der Ausgaserzone (A) genutzt wird,
**dadurch gekennzeichnet, dass** aus der Ausgaserzone (A) von einer Trennstufe, die eine oder mehrere Trennstufen unterhalb der Zuführung des Sumpfstroms (LM/C₄H₈) aus der Waschzone (E) liegt, die Flüssigkeit oder ein Teilstrom der Flüssigkeit aus der Ausgaserzone (A) abgezogen, durch indirekten Wärmeaustausch mit dem heißen Sumpfstrom (LM) aus der Ausgaserzone (A) erwärmt und/oder verdampft und auf derselben Trennstufe oder oberhalb derselben in die Ausgaserzone A zurückgeführt wird, wobei die Trennstufe, von der die Flüssigkeit oder der Flüssigkeitsteilstrom abgezogen wird, dergestalt gewählt wird, dass der Gesamtenergiebedarf in den Verfahrensstufen I und II minimal ist.

2. Verfahren nach Anspruch 1, **dadurch gekennzeichnet, dass** der C₄-Schnitt (C₄) der Waschzone (E) gasförmig, bevorzugt im unteren Bereich derselben, zugeführt wird.

3. Verfahren nach Anspruch 1 oder 2, **dadurch gekennzeichnet, dass** man aus der Waschzone (E) von einer Trennstufe, die eine oder mehrere Trennstufen unterhalb der Zuführung des Stromes des selektiven Lösungsmittels (LM), bevorzugt unterhalb der Zuführung des C₄-Schnittes (C₄), liegt, die Flüssigkeit oder einen Teilstrom der Flüssigkeit abzieht, durch indirekten Wärmeaustausch mit dem heißen Sumpfstrom (LM) aus der Ausgaserzone (A) erwärmt und/oder verdampft und auf derselben Trennstufe oder oberhalb derselben in die Waschzone (E) zurückführt, wobei man die Trennstufe, von der man die Flüssigkeit oder der Flüssigkeitsteilstrom abzieht, dergestalt wählt, dass der Gesamtenergiebedarf in den Verfahrensstufen I und II minimal ist.

4. Verfahren nach einem der Ansprüche 1 bis 3, **dadurch gekennzeichnet, dass** man als selektives Lösungsmittel eine oder mehrere der Substanzen N-Methylpyrrolidon (NMP), Dimethylformamid, Acetonitril und Furfurol oder Mischungen einer oder mehrerer der vorstehend genannten Substanzen mit Colösungsmitteln einsetzt.

5. Verfahren nach Anspruch 4, **dadurch gekennzeichnet, dass** man NMP mit 0 bis 20 Gew.-% Wasser, insbesondere mit 7 bis 10 Gew.-% Wasser, besonders bevorzugt mit 8,3 Gew.-% Wasser, einsetzt.

6. Verfahren nach einem der Ansprüche 1 bis 5, **dadurch gekennzeichnet, dass** die Rückführung der Flüssigkeit oder des Teilstroms der Flüssigkeit aus der Ausgaserzone (A) (und/oder aus der Waschzone (E)) auf dieselbe Trennstufe erfolgt, von der die Flüssigkeit oder der Teilstrom der Flüssigkeit abgezogen wurde.

7. Verfahren nach einem der Ansprüche 1 bis 6, **dadurch gekennzeichnet, dass** man abgezogenen Flüssigkeitsstrom oder -teilstrom einer Entspannungsverdampfung unter Erhalt einer dampfförmigen und einer flüssigen Phase unterwirft und anschließend die dampfförmige und die flüssige Phase auf dieselbe Stufe zurückführt von der der Flüssigkeitsstrom oder -teilstrom abgezogen wurde oder dass man den dampfförmigen Anteil des abgezogenen Flüssigkeitsstromes oder - teilstromes eine oder mehrere Stufen oberhalb der Stufe zuführt, von der der Flüssigkeitsstrom oder -teilstrom abgezogen wurde.

8. Verfahren nach einem der Ansprüche 1 bis 7, **gekennzeichnet durch** eine Anzahl von theoretischen Trennstufen von 10 bis 80, bevorzugt von 20 bis 30, insbesondere von 26 in der Waschzone (E) und von 1 bis 30, bevorzugt von 2 bis 8, insbesondere von 4, in der Ausgaserzone (A).

9. Verfahren nach einem der Ansprüche 1 bis 8, **dadurch gekennzeichnet, dass** die Waschzone (E) und die Ausgaserzone (A) in einer einzigen Kolonne angeordnet sind.

## Claims

1. A continuous process for isolating butenes from a C₄ fraction comprising butanes, butenes and possibly traces of other hydrocarbons by extractive distillation using a selective solvent (LM), in which
the C₄ fraction is, in a first process stage I, separated in a scrubbing zone (E) into which the C₄ fraction (C₄) is fed in gaseous form or liquid form and the selective solvent (LM) is fed in liquid form above the feed point for the C₄ fraction (C₄) into a butane-comprising top stream (C₄H₁₀) and a bottom stream (LM/C₄H₈) comprising the selective solvent laden with the butenes and possibly the traces of other hydrocarbons, and the bottom stream (LM/C₄H₈) is,
in a second process stage II, separated in a degassing zone (A) to which energy is fed via a bottom vaporizer (W5) and which is at a higher temperature and if appropriate lower pressure than the scrubbing zone (E) into a top stream (C₄H₈) comprising the butenes and any traces of other hydrocarbons and a bottom stream (LM) comprising the selective solvent, with the heat of the bottom stream from the degassing zone (A) being utilized for increasing the temperature in the degassing zone (A),
wherein the liquid or a substream of the liquid is taken off from the degassing zone (A) at a theoretical plate located one or more theoretical plates below the feed point for the bottom stream (LM/C₄H₈) from the scrubbing zone (E), heated and/or vaporized by indirect heat exchange with the hot bottom stream (LM) from the degassing zone (A) and returned to the degassing zone (A) at the same theoretical plate or above this, with the theoretical plate from which the liquid or substream of liquid is taken off being selected so that the total energy requirement in the process stages I and II is minimized.

2. The process according to claim 1, wherein the C₄ fraction (C₄) is fed in gaseous form to the scrubbing zone (E), preferably in the lower region of the latter.

3. The process according to claim 1 or 2, wherein the liquid or a substream of the liquid is taken off from the scrubbing zone (E) at a theoretical plate located one or more theoretical plates below the feed point for the stream of the selective solvent (LM), preferably below the feed point for the C₄ fraction (C₄), heated and/or vaporized by indirect heat exchange with the hot bottom stream (LM) from the degassing zone (A) and returned to the scrubbing zone (E) at the same theoretical plate or above this, with the theoretical plate from which the liquid or the substream of liquid is taken off being selected so that the total energy requirement in the process stages I and II is minimized.

4. The process according to any of claims 1 to 3, wherein the selective solvent used consists of one or more of the substances N-methylpyrrolidone
(NMP), dimethylformamide, acetonitrile and furfural or a mixture of one or more of the abovementioned substances with cosolvents.

5. The process according to claim 4, wherein NMP containing from 0 to 20% by weight of water, in particular from 7 to 10% by weight of water, particularly preferably 8.3% by weight of water, is used.

6. The process according to any of claims 1 to 5, wherein the liquid or the substream of the liquid from the degassing zone (A) (and/or from the scrubbing zone (E)) is returned to the same theoretical plate from which the liquid or the substream of the liquid was taken off.

7. The process according to any of claims 1 to 6, wherein liquid stream or substream taken off is subjected to expansion evaporation to give a gaseous phase and a liquid phase and the gaseous and liquid phases are subsequently returned to the same theoretical plate from which the liquid stream or substream was taken off or the gaseous part of the liquid stream or substream which was taken off is fed to one or more theoretical plates above the theoretical plate from which the liquid stream or substream was taken off.

8. The process according to any of claims 1 to 7, wherein the number of theoretical plates in the scrubbing zone (E) is from 10 to 80, preferably from 20 to 30, in particular 26, and the number of theoretical plates in the degassing zone (A) is from 1 to 30, preferably from 2 to 8, in particular 4.

9. The process according to any of claims 1 to 8, wherein the scrubbing zone (E) and the degassing zone (A) are both located in a single column.

## Revendications

1. Procédé continu d'obtention de butènes à partir d'une coupe en C₄ contenant des butanes, des butènes et éventuellement des traces d'autres hydrocarbures par distillation extractive avec un solvant sélectif (LM), selon lequel
lors d'une première étape de procédé I dans une zone de lavage (E), qui est alimentée avec la coupe en C₄ (C₄) sous forme gazeuse ou liquide et le solvant sélectif (LM) sous forme liquide au-dessus de l'alimentation de la coupe en C₄ (C₄), la coupe en C₄ est séparée en un courant de tête (C₄H₁₀) contenant les butanes et un courant de fond (LM/C₄H₈) contenant le solvant sélectif chargé avec les butènes et éventuellement les traces d'autres hydrocarbures, et selon lequel le courant de fond (LM/C₄H₈)
lors d'une seconde étape de procédé II dans une zone de dégazage (A), qui est alimentée en énergie par un évaporateur de fond (W5), est séparé à température élevée et éventuellement à pression réduite par rapport à la zone de lavage (E) en un courant de tête (C₄H₈) contenant les butènes et éventuellement des traces d'autres hydrocarbures et un courant de fond (LM) contenant le solvant sélectif, la chaleur du courant de fond issu de la zone de dégazage (A) étant utilisée pour l'élévation de température dans la zone de dégazage (A),
**caractérisé en ce que** le liquide ou un courant partiel du liquide de la zone de dégazage (A) est soutiré de la zone de dégazage (A) à partir d'une étape de séparation située une ou plusieurs étapes de séparation en dessous de l'alimentation du courant de fond (LM/C₄H₈) issu de la zone de lavage (E), chauffé et/ou évaporé par échange thermique indirect avec le courant de fond (LM) chaud issu de la zone de dégazage (A) et recyclé dans la même étape de séparation ou au-dessus de celle-ci dans la zone de dégazage (A), l'étape de séparation à partir de laquelle le liquide ou le courant partiel de liquide est soutiré étant choisie de manière à ce que le besoin énergétique total dans les étapes de procédé I et II soit minimal.

2. Procédé selon la revendication 1, **caractérisé en ce que** la coupe en C₄ (C₄) est introduite sous forme gazeuse dans la zone de lavage (E), de préférence dans sa partie inférieure.

3. Procédé selon la revendication 1 ou 2, **caractérisé en ce que** le liquide ou un courant partiel du liquide est soutiré de la zone de lavage (E) à partir d'une étape de séparation située une ou plusieurs étapes de séparation en dessous de l'alimentation du courant du solvant sélectif (LM), de préférence en dessous de l'alimentation de la coupe en C₄ (C₄, chauffé et/ou évaporé par échange thermique indirect avec le courant de fond (LM) chaud issu de la zone de dégazage (A) et recyclé dans la même étape de séparation ou au-dessus de celle-ci dans la zone de lavage (E), l'étape de séparation à partir de laquelle le liquide ou le courant partiel de liquide est soutiré étant choisie de manière à ce que le besoin énergétique total dans les étapes de procédé I et II soit minimal.

4. Procédé selon l'une quelconque des revendications 1 à 3, **caractérisé en ce qu'**une ou plusieurs des substances N-méthylpyrrolidone (NMP), diméthylformamide, acétonitrile et furfurol ou des mélanges d'une ou de plusieurs des substances susmentionnées avec des co-solvants sont utilisés en tant que solvant sélectif.

5. Procédé selon la revendication 4, **caractérisé en ce que** de la NMP contenant 0 à 20 % en poids d'eau, notamment 7 à 10 % en poids d'eau, 1 de manière particulièrement préférée 8,3 % en poids d'eau, est utilisée.

6. Procédé selon l'une quelconque des revendications 1 à 5, **caractérisé en ce que** le recyclage du liquide ou du courant partiel du liquide issu de la zone de dégazage (A) (et/ou issu de la zone de lavage (E)) a lieu dans la même étape de séparation que celle à partir de laquelle le liquide ou le courant partiel du liquide a été soutiré.

7. Procédé selon l'une quelconque des revendications 1 à 6, **caractérisé en ce que** le courant ou courant partiel de liquide soutiré est soumis à une évaporation avec détente, avec obtention d'une phase gazeuse et d'une phase liquide, puis la phase gazeuse et la phase liquide sont réintroduites dans la même étape que celle à partir de laquelle le courant ou courant partiel de liquide a été soutiré, ou la fraction gazeuse du courant ou courant partiel de liquide soutiré est introduite une ou plusieurs étapes au-dessus de l'étape à partir de laquelle le courant ou courant partiel de liquide a été soutiré.

8. Procédé selon l'une quelconque des revendications 1 à 7, **caractérisé par** un nombre d'étapes de séparation théoriques de 10 à 80, de préférence de 20 à 30, notamment de 26, dans la zone de lavage (E) et de 1 à 30, de préférence de 2 à 8, notamment de 4, dans la zone de dégazage (A).

9. Procédé selon l'une quelconque des revendications 1 à 8, **caractérisé en ce que** la zone de lavage (E) et la zone de dégazage (A) sont placées dans une seule colonne.
